(19) Europäisches Patentamt / European Patent Office / Office européen des brevets

(11) **EP 4 663 718 A1**

(12) **EUROPEAN PATENT APPLICATION**
published in accordance with Art. 153(4) EPC

(43) Date of publication:
**17.12.2025 Bulletin 2025/51**

(21) Application number: **24792350.1**

(22) Date of filing: **19.02.2024**

(51) International Patent Classification (IPC):
***C10B 57/00*** (2006.01)  ***C10B 57/04*** (2006.01)

(52) Cooperative Patent Classification (CPC):
**C10B 57/00; C10B 57/04**

(86) International application number:
**PCT/JP2024/005707**

(87) International publication number:
**WO 2024/219074 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.04.2023 JP 2023069154**

(71) Applicant: JFE Steel Corporation
**Tokyo 100-0011 (JP)**

(72) Inventors:
• **ARAKAWA, Sara**
**Tokyo 100-0011 (JP)**
• **YAMAMOTO, Tetsuya**
**Tokyo 100-0011 (JP)**
• **TANDOKORO, Kohei**
**Tokyo 100-0011 (JP)**
• **ISHIDA, Tomoharu**
**Tokyo 100-0011 (JP)**

(74) Representative: Hoffmann Eitle
**Patent- und Rechtsanwälte PartmbB**
**Arabellastraße 30**
**81925 München (DE)**

(54) **METHOD FOR ESTIMATING BLENDED COAL COKE STRENGTH AFTER REACTION, METHOD FOR PRODUCING BLENDED COAL COKE, METHOD FOR OPERATING COKE OVEN, AND METHOD FOR CONTROLLING SINGLE COAL BLENDING RATIO**

(57) A method for readily estimating the coke strength of coal-blend coke after $CO_2$ reaction is provided.

A coal-blend coke CSR estimating method for estimating the coke strength of coal-blend coke after $CO_2$ reaction (CSR) using a TG reactivity index that is a percentage weight loss measured by thermogravimetry of coke held in a $CO_2$ atmosphere at a predetermined temperature range for a predetermined time, the method including a step of measuring the TG reactivity indices of single-coal cokes and estimating the TG reactivity index of the corresponding coal-blend coke from the weighted average of the TG reactivity indices measured of the single-coal cokes; a step of estimating the coke reactivity index (CRI) of the coal-blend coke from the TG reactivity index of the coal-blend coke estimated in the previous step; and a step of estimating the coke strength of the coal-blend coke after $CO_2$ reaction (CSR) from the coke reactivity index (CRI) of the coal-blend coke estimated in the previous step and the drum index (DI) of the coal-blend coke.

FIG. 4

FIG. 4 — Plot of ACTUALLY MEASURED COKE STRENGTH OF COAL-BLEND COKE AFTER $CO_2$ REACTION (CSR) [-] (y-axis, 50–75) versus ESTIMATED COKE STRENGTH OF COAL-BLEND COKE AFTER $CO_2$ REACTION (CSR) [-] (x-axis, 50–75).

**Description**

Technical Field

The present invention relates to a method for readily estimating the coke strength of coal-blend coke after $CO_2$ reaction.

Background Art

Blast furnaces are traditionally charged with iron raw materials, such as iron ores, and also cokes as auxiliary raw materials. In order to produce coke of desired quality, coals with different qualities are mixed to adjust the grade and the resultant coal blend is pulverized and carbonized in a coke oven. The coals (coal brands) before mixing are called single coals.

Since the quality of coke has a significant effect on blast furnace operation, stable production of high-quality coke is necessary. In particular, coke has a role in ensuring gas permeability in a blast furnace. It is therefore important to produce high-strength coke in order to eliminate or reduce the generation of a powder that will inhibit the passage of gas in the furnace. The impact applied to coke varies from location to location in a blast furnace. Thus, the coke strength is managed using strength indices, such as drum index (DI) and coke strength after $CO_2$ reaction (CSR).

In particular, the coke strength after $CO_2$ reaction (CSR) is used as the strength index of the susceptibility of coke to deterioration by hot reaction with $CO_2$ in a blast furnace. The measurement of CSR is specified in ISO 18894 and is defined as follows. 200 g of coke having particle sizes adjusted to $20 \pm 1$ mm is reacted at 1100°C for 2 hours while flowing 100% $CO_2$ gas at 5 L/min. The reacted coke is treated for 600 revolutions in an I-type drum tester and is sieved through a 9.56 mm sieve. The mass yield of the coke remaining on the sieve is taken as CSR. The percentage weight loss of the coke before and after the reaction at 1100°C for 2 hours is specified as the coke reactivity index (CRI) in ISO 18894.

Owing to measurement technique characteristics, the coke strength after $CO_2$ reaction (CSR) can be estimated from the drum index (DI) of the coke before reaction and the coke reactivity index (CRI). CSR has a particularly high correlation with CRI. Thus, estimating the coke reactivity index (CRI) of coal-blend coke is important in order to estimate the coke strength of the coal-blend coke after $CO_2$ reaction (CSR).

Patent Literature 1 proposes a technique for estimating the coke strength of coal-blend coke after $CO_2$ reaction. In the technique, the weighted average of the CSRs of single coals multiplied by the blending ratios is corrected based on coal blending effects, specifically, the overlap of the thermoplastic regions of the coals measured by the Gieseler Plastometer method, the total average reflectance, and the maximum fluidity.

Furthermore, Patent Literature 2 proposes a technique for estimating the coke reactivity index of coal-blend coke. In the technique, the coke reactivity indices of single-coal cokes are multiplied by the blending ratios of the respective single coals and the resultant weighted average is corrected with the specific volume.

Patent Literatures 3 and 4 propose techniques for estimating the CSR of coal-blend coke from the CRI of the coal-blend coke calculated as the weighted average of the CRIs of the single-coal cokes, and the cold surface fracture strength $DI^{150}_6$.

Citation List

Patent Literature

PTL 1: Japanese Unexamined Patent Application Publication No. 63-199286
PTL 2: Japanese Unexamined Patent Application Publication No. 9-263766
PTL 3: Japanese Unexamined Patent Application Publication No. 2005-232350
PTL 4: Japanese Unexamined Patent Application Publication No. 2013-1895

Summary of Invention

Technical Problem

Many of the above techniques for estimating the coke strength of coal-blend coke after $CO_2$ reaction (CSR) assume that the CSRs and the CRIs of single-coal cokes have been measured. However, the measurements of CSR and CRI take a long time. Due to this fact, the estimation of the coke strength of coal-blend coke after $CO_2$ reaction (CSR) has a drawback in that an extended time is required to prepare single-coal cokes from all the coal brands that have come in stock and to measure the CSR and the CRI of each single-coal coke. Furthermore, the CRI is affected by other factors, such as residual volatile matter (VM) in the coke, and thus the measurement results may include variations.

An object of the present invention is to provide a method for readily estimating the coke strength of coal-blend coke after $CO_2$ reaction.

Solution to Problem

The present invention is directed to estimating the coke strength of coal-blend coke after $CO_2$ reaction (CSR) readily and simply by using thermogravimetry. The present invention has the following configurations.

[1] A coal-blend coke CSR estimating method for estimating the coke strength of coal-blend coke after $CO_2$ reaction (CSR) using a TG reactivity index that is a percentage weight loss measured by thermogravimetry of coke held in a $CO_2$ atmosphere at a predetermined temperature range for a predetermined time, the method including:

a step of measuring the TG reactivity indices of single-coal cokes and estimating the TG reactivity index of the corresponding coal-blend coke from the weighted average of the TG reactivity indices measured of the single-coal cokes;
a step of estimating the coke reactivity index (CRI) of the coal-blend coke from the TG reactivity index of the coal-blend coke estimated in the previous step; and
a step of estimating the coke strength of the coal-blend coke after $CO_2$ reaction (CSR) from the coke reactivity index (CRI) of the coal-blend coke estimated in the previous step and the drum index (DI) of the coal-blend coke.

[2] The coal-blend coke CSR estimating method according to [1], wherein the thermogravimetry is performed by heating the coke in an inert gas atmosphere to a temperature range from 950°C to 1100°C.
[3] The coal-blend coke CSR estimating method according to [1] or [2], wherein the predetermined time is 10 minutes or more and 120 minutes or less.
[4] A method for producing coal-blend coke, including:

estimating the coke strength of coal-blend coke after $CO_2$ reaction using the coal-blend coke CSR estimating method described in any one of [1] to [3]; and
changing a production condition based on the estimated coke strength of coal-blend coke after $CO_2$ reaction.

[5] A method of operating a coke oven, including:

estimating the coke strength of coal-blend coke after $CO_2$ reaction using the coal-blend coke CSR estimating method described in any one of [1] to [3]; and
changing a coke oven operation condition based on the estimated coke strength of coal-blend coke after $CO_2$ reaction.

[6] A method for controlling the blending ratios of single coals, including:

estimating the coke strength of coal-blend coke after $CO_2$ reaction using the coal-blend coke CSR estimating method described in any one of [1] to [3]; and
changing the blending ratios of single coals based on the estimated coke strength of coal-blend coke after $CO_2$ reaction.

Advantageous Effects of Invention

The present invention enables fast estimation of the coke strength of coal-blend coke after $CO_2$ reaction (CSR).
According to the present invention, the percentage weight losses (TG reactivity indices) of single-coal cokes are measured by thermogravimetry, and the TG reactivity indices are weighted-averaged to give the TG reactivity index of the corresponding coal-blend coke, which is then converted into the coke reactivity index (CRI) of the coal-blend coke. After the coke reactivity index (CRI) of the coal-blend coke is thus determined, the coke strength of the coal-blend coke after $CO_2$ reaction (CSR) is estimated from the coke reactivity index (CRI) of the coal-blend coke and the drum index (DI) of the coal-blend coke. In this manner, the coke strength of coal-blend coke after $CO_2$ reaction can be readily estimated.

Brief Description of Drawings

[Fig. 1] Fig. 1 is a graph illustrating relationships between the weighted average of actually measured TG reactivity indices of single-coal cokes and the actually measured TG reactivity index of the corresponding coal-blend coke.
[Fig. 2] Fig. 2 is a graph illustrating relationships between the estimated TG reactivity index of coal-blend coke and the actually measured coke reactivity index (CRI) of the coal-blend coke.
[Fig. 3] Fig. 3 is a graph illustrating relationships between the coke strength after $CO_2$ reaction (CSR) and the coke reactivity index (CRI) for various coke drum indices (DI).
[Fig. 4] Fig. 4 is a graph illustrating relationships between the estimated coke strength of coal-blend coke after $CO_2$ reaction (CSR) and the actually measured coke strength of the coal-blend coke after $CO_2$ reaction (CSR).

Description of Embodiments

Embodiments of the present invention will be described below. However, the present invention is not limited to the following embodiments.

As already mentioned, the coke strength after $CO_2$ reaction (CSR) is governed by the coke reactivity index (CRI) and the drum index (DI) of the coke. Thus, the coke strength of coal-blend coke after $CO_2$ reaction (CSR) can be estimated if the coke reactivity index (CRI) of the coal-blend coke and the drum index (DI) of the coal-blend coke are available.

First, a process for estimating the coke reactivity index (CRI) of coal-blend coke will be described. In the present embodiment, the coke reactivity index (CRI) of coal-blend coke is estimated through the following steps. The first step measures the percentage weight losses of single-coal cokes (the TG reactivity indices of single-coal cokes) by thermogravimetry in which the single-coal cokes are held in a $CO_2$ atmosphere at a predetermined temperature range for a predetermined time. The next step estimates the TG reactivity index of the corresponding coal-blend coke from the weighted average of the TG reactivity indices of the single-coal cokes measured in the previous step. The subsequent step estimates the coke reactivity index (CRI) of the coal-blend coke from the TG reactivity index of the coal-blend coke estimated in the previous step. These steps will be individually described below.

[Step of measuring the percentage weight losses of single-coal cokes (the TG reactivity indices of single-coal cokes)]

This step utilizes thermogravimetry (TG). Thermogravimetry is capable of measuring the over-time change in weight (the percentage weight loss) stemming from the reaction of carbon in coke with $CO_2$ in a high-temperature 100% $CO_2$ atmosphere. Because the weight loss of coke due to this reaction is linear, it is possible to estimate the coke reactivity index (CRI) of coal-blend coke, which will be described later, even when the reaction time (the measurement time) is short.

In the present specification, the percentage weight loss measured by thermogravimetry in which coke is held in a $CO_2$ atmosphere at a predetermined temperature range for a predetermined time is also written as the TG reactivity index.

In this step, the percentage weight losses of single-coal cokes (the TG reactivity indices of single-coal cokes) are measured by TG in which the single-coal cokes are held in a $CO_2$ atmosphere at a predetermined temperature range for a predetermined time. The TG measurement is performed with a TG device. The single-coal cokes used as TG samples may be cokes obtained by carbonizing single coals. For example, single-coal cokes prepared by carbonization test in cans may be used, or small amounts of samples obtained by sample division may be carbonized under predetermined conditions to give single-coal cokes. The TG samples may be sampled in any manner without limitation. The amount of sample is limited by the specifications of the TG device. In order to enhance the measurement accuracy, the sampling preferably ensures that the sample will show representative properties of the single-coal coke. For example, it is preferable that the sampling conform to "Coal and coke - Sampling and sample preparation" in JIS M 8811: 2000.

The amount of sample to be subjected to the TG measurement may be adjusted to the capacity and the size of the TG device. It is preferable that the particle size of the TG sample be determined so that the sample contains a certain number of particles enough to have representativeness. From this viewpoint, the particle size of single-coal coke used as the sample is preferably 850 $\mu$m or less. The particle size may be controlled by pulverizing the single-coal coke. Furthermore, the specific surface area of the sample is influential to the reaction with $CO_2$ and, in particular, the amount of fine particles contained in the sample greatly affects the specific surface area of the sample. It is therefore preferable to remove fine particles with a particle size of less than 200 $\mu$m. From the above viewpoints, the particle size of the sample is preferably 200 $\mu$m or more and 850 $\mu$m or less. The particle size of the TG sample may be controlled by sieving the pulverized single-coal coke through a sieve with a predetermined mesh size.

Next, the TG measurement conditions will be described. The TG measurement is performed as follows. The sample is set on the TG device and is heated to a predetermined temperature range in an inert gas atmosphere, such as nitrogen. The sample is then held in a $CO_2$ atmosphere for a predetermined time. Specifically, after the sample has been heated to a predetermined temperature range in an inert gas atmosphere, the gas supplied to the TG device is switched to $CO_2$ gas, and the sample is held in a $CO_2$ atmosphere for a predetermined time and is then TG analyzed. The heating rate in the above process may be controlled to the capacity of the TG device. A higher heating rate leads to a shorter measurement time and is therefore more preferable. From this viewpoint, the heating rate is preferably 20°C/min or more. The upper limit of the heating rate is not particularly limited and is such a heating rate that the heating process will not exceed the temperature control ability of the device and will not raise the temperature above the predetermined temperature range. As an example, the heating rate may be 150°C/min or less. The $CO_2$ gas feed rate is preferably enough to ensure that the supply of $CO_2$ gas during the reaction will not limit the reaction rate. The $CO_2$ gas feed rate that will not limit the reaction rate varies depending on the amount of sample and the size of TG device. It is therefore preferable to determine beforehand the gas feed rate that is required for the actual TG device. For example, the $CO_2$ gas feed rate is preferably 500 mL/min or more in the case of a general TG device for analyzing approximately several tens of mg of a measurement sample.

From the point of view focused on the reactivity of the matrix of the coke, the predetermined temperature range (the temperatures of the reaction with $CO_2$) is preferably from 950°C to 1100°C in order to eliminate disturbance influences, such as the influence of pore structures.

From the point of view of the reactivity with $CO_2$, the predetermined time is preferably 10 minutes or more. From the point of view of shortening the measurement time, the predetermined time is preferably 120 minutes or less. As already described, the predetermined time is a holding time in the $CO_2$ atmosphere at the predetermined temperature range.

After the single-coal coke has been held in a $CO_2$ atmosphere at a predetermined temperature range for a predetermined time as described above, the percentage weight loss (the TG reactivity index of the single-coal coke) is measured.

[Step of estimating the TG reactivity index of coal-blend coke]

The TG reactivity indices of single-coal cokes measured by TG have additivity. It is therefore possible to estimate the TG reactivity index of coal-blend coke from the weighted average of the TG reactivity indices of single-coal cokes multiplied by the blending ratios of the single coals constituting the coal blend. In other words, the TG reactivity index of coal-blend coke after being held in a $CO_2$ atmosphere at a predetermined temperature range for a predetermined time can be estimated from the measured TG reactivity indices of the single-coal cokes.

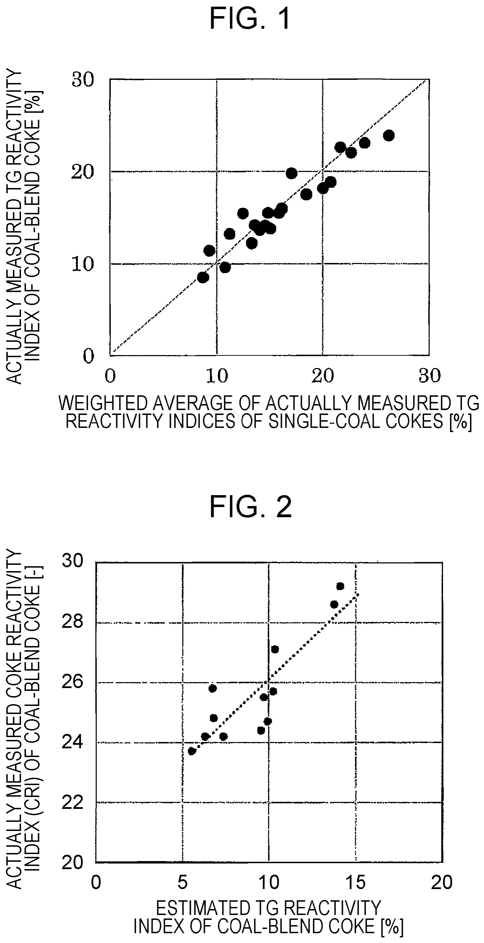

Fig. 1 plots the weighted average of actually measured TG reactivity indices of single-coal cokes on the horizontal axis and the actually measured TG reactivity index of the corresponding coal-blend coke on the vertical axis. As illustrated in Fig. 1, there is a correlation between the weighted average of actually measured TG reactivity indices of single-coal cokes and the actually measured TG reactivity index of the corresponding coal-blend coke. In other words, the results show that the TG reactivity index of coal-blend coke after blending can be estimated based on the TG reactivity indices of single-coal cokes. This step uses this correlation in estimating the TG reactivity index of the coal-blend coke from the weighted average of the actually measured TG reactivity indices of the single-coal cokes.

[Step of estimating the coke reactivity index (CRI) of the coal-blend coke]

In this step, the coke reactivity index (CRI) of the coal-blend coke is estimated from the TG reactivity index of the coal-blend coke estimated in the previous step. Fig. 2 illustrates relationships between the estimated TG reactivity index of coal-blend coke and the actually measured coke reactivity index (CRI) of the coal-blend coke. As illustrated in Fig. 2, there is a correlation between the estimated TG reactivity index of coal-blend coke and the actually measured coke reactivity index (CRI) of the coal-blend coke. This correlation can give in advance an expression for estimating the coke reactivity index (CRI) of coal-blend coke (expression (1) described later). The coke reactivity index (CRI) of coal-blend coke can be thus estimated from the estimated TG reactivity index of the coal-blend coke.

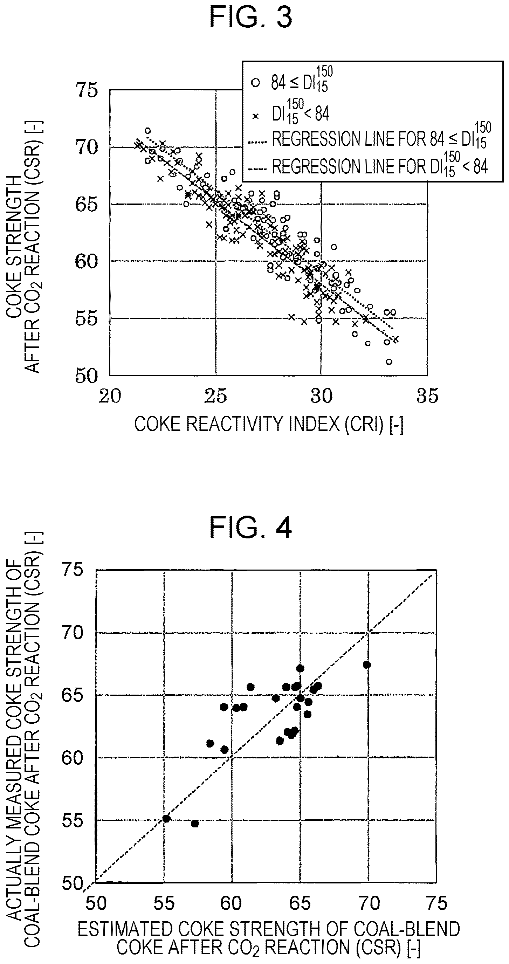

Next, a process for estimating the coke strength of the coal-blend coke after $CO_2$ reaction (CSR) from the estimated coke reactivity index (CRI) of the coal-blend coke and the drum index (DI) of the coal-blend coke will be described. In the present embodiment, the drum index (DI) of the coal-blend coke is $DI^{150}_{15}$ specified in JIS K 2151: 2004. However, the drum index (DI) of the coal-blend coke is not limited thereto and may be, for example, a value obtained from a known estimation expression. Fig. 3 illustrates relationships between the coke strength after $CO_2$ reaction (CSR) and the coke reactivity index (CRI) for various coke drum indices ($DI^{150}_{15}$), plotting actually measured values of the coke reactivity index (CRI) on the horizontal axis and actually measured values of the coke strength after $CO_2$ reaction (CSR) on the vertical axis. Fig. 3 shows that there is a strong correlation between the coke strength after $CO_2$ reaction (CSR) and the coke reactivity index (CRI), and further shows that the variation in coke strength after $CO_2$ reaction (CSR) at the same coke reactivity index (CRI) is affected by the coke drum index (DI). By utilizing this relationship, it is possible to estimate the coke strength after $CO_2$ reaction (CSR) from the coke reactivity index (CRI) and the coke drum index (DI). In the present embodiment, the coke strength of the coal-blend coke after $CO_2$ reaction (CSR) can be readily estimated using the coke reactivity index (CRI) of the coal-blend coke estimated by the above-described process.

In the present invention, the TG measurement of the reactivity of cokes from single coals to be blended enables estimating the coke strength after $CO_2$ reaction (CSR) that will be obtained when the single coals are blended and carbonized into the corresponding coal-blend coke. Thus, the present invention allows for fast estimation of the coke strength of coal-blend coke after $CO_2$ reaction (CSR).

The coal-blend coke CSR estimating method of the present invention allows for coke production in a way that the blending ratios of coals (single coals) are controlled while estimating the coke strength after $CO_2$ reaction (CSR) that will be obtained when the single coals are blended and carbonized into the corresponding coal-blend coke. Thus, coal-blend coke with stable CSR quality can be produced. Furthermore, the capability of producing coal-blend coke while estimating the coke strength of the coal-blend coke after $CO_2$ reaction (CSR) can reduce variations in CSR. Thus, for example, it becomes possible to design the blending more closely to the lower limit of the target CSR range than heretofore possible, to expand the range of raw material grades that can be used, and to reduce the costs for the production of coal-blend coke.

A method for producing coal-blend coke of the present invention uses the coal-blend coke CSR estimating method described hereinabove. Specifically, the coke strength of coal-blend coke after $CO_2$ reaction is estimated using the above coal-blend coke CSR estimating method, and production conditions are changed based on the estimated coke strength of coal-blend coke after $CO_2$ reaction. More specifically, production conditions may be changed so that the estimated coke strength of coal-blend coke after $CO_2$ reaction will reach a target coke strength. For example, the production conditions include the blending ratios (the blending percentages) of coals (single coals).

A method of operating a coke oven according to the present invention uses the coal-blend coke CSR estimating method described hereinabove. Specifically, the coke strength of coal-blend coke after $CO_2$ reaction is estimated using the above coal-blend coke CSR estimating method, and coke oven operation conditions are changed based on the estimated coke strength of coal-blend coke after $CO_2$ reaction. More specifically, coke oven operation conditions may be changed so that the estimated coke strength of coal-blend coke after $CO_2$ reaction will reach a target coke strength. For example, the operation conditions include the blending ratios of coals (single coals).

A method for controlling the blending ratios of single coals according to the present invention uses the coal-blend coke CSR estimating method described hereinabove. Specifically, the coke strength of coal-blend coke after $CO_2$ reaction is estimated using the above coal-blend coke CSR estimating method, and the blending ratios of single coals are changed based on the estimated coke strength of coal-blend coke after $CO_2$ reaction. More specifically, the blending ratios of single coals may be changed so that the estimated coke strength of coal-blend coke after $CO_2$ reaction will reach a target coke strength.

The method for producing coal-blend coke, the method of operating a coke oven, and the method for controlling the blending ratios of single coals according to the present invention can achieve the same effects as described hereinabove by virtue of the use of the coal-blend coke CSR estimating method of the present invention.

EXAMPLES

Hereinbelow, the present invention will be described based on Examples. However, the present invention is not limited to the following Examples.

Coal-blend cokes ready for being charged into an actual furnace were measured for the coke strength of the coal-blend coke after $CO_2$ reaction (CSR) and the coke reactivity index (CRI) of the coal-blend coke in accordance with ISO 18894, and were also measured for the drum index ($DI^{150}_{15}$) of the coal-blend coke in accordance with JIS K 2151: 2004. The single coals that were used in this coke production were tested in accordance with JIS M 8812: 2006 to measure volatile matters (VM), and the resultant carbides (single-coal cokes) were sampled. Each of the single-coal coke samples was pulverized to 850 $\mu$m or less, and sample division was performed. The particles were then sieved through a 200 $\mu$m sieve. The grain size was thus controlled to 850 $\mu$m to 200 $\mu$m.

The TG reactivity index of the single-coal cokes was measured with TG device STA1600 manufactured by LINSEIS. The sample weight was 0.5 g. In a $N_2$ atmosphere, the sample was heated to 950°C at 100°C/min and was held for 20 minutes until the temperature atmosphere became stabilized. Subsequently, the gas was switched from $N_2$ to $CO_2$. The $CO_2$ gas feed rate was set to 1000 mL/min, and the sample was held (reacted) in a $CO_2$ atmosphere for 60 minutes. The percentage weight loss [%] was measured from [(weight before reaction - weight after reaction)/(weight before reaction)] $\times$ 100. The weighted average of the percentage weight losses (the TG reactivity indices) of the single-coal cokes multiplied by the blending ratios of the single-coal cokes was calculated to estimate the TG reactivity index of the coal-blend coke.

Next, the correlation illustrated in Fig. 2 between the estimated TG reactivity index of coal-blend coke and the actually measured coke reactivity index (CRI) of the coal-blend coke was studied, and expression (1) described below was established for the estimation of the coke reactivity index (CRI) of coal-blend coke.

Coke reactivity index (CRI) of coal-blend coke = a $\times$ (TG reactivity index of coal-blend coke) + b       (1)

Here, a and b in expression (1) are constants. In this Example, a = 0.55 and b = 20. The TG reactivity index of the coal-blend coke estimated above was substituted into expression (1) to determine the coke reactivity index (CRI) of the coal-blend coke.

The relationships between the coke strength after $CO_2$ reaction (CSR) and the coke reactivity index (CRI) according to ISO 18894 for various coke drum indices (DI, $DI^{150}_{15}$ in this Example) are as illustrated in Fig. 3. From the relationships, expression (2) described below was established as the regression expression for estimating the coke strength after $CO_2$ reaction (CSR).

Coke strength after $CO_2$ reaction (CSR) = c × (coke reactivity index (CRI)) + d × (coke $DI^{150}_{15}$) + e          (2)

Here, c, d, and e are constants. In this Example, c = -1.4, d = 0.91, and e = 24. The coke reactivity index (CRI) of the coal-blend coke determined from expression (1), and the actually measured drum index ($DI^{150}_{15}$) of the coal-blend coke were substituted into expression (2) to determine the coke strength of the coal-blend coke after $CO_2$ reaction (CSR). The relationships between the thus-estimated coke strength of the coal-blend coke after $CO_2$ reaction (CSR) and the actually measured coke strength of the coal-blend coke after $CO_2$ reaction (CSR) are illustrated in Fig. 4. As illustrated in Fig. 4, the coal-blend coke CSR estimating method of the present invention, based on the TG reactivity indices of single-coal cokes measured by TG, can estimate the coke strength of the corresponding coal-blend coke after $CO_2$ reaction (CSR).

**Claims**

1. A coal-blend coke CSR estimating method for estimating a coke strength of coal-blend coke after $CO_2$ reaction (CSR) using a TG reactivity index that is a percentage weight loss measured by thermogravimetry of coke held in a $CO_2$ atmosphere at a predetermined temperature range for a predetermined time, the method comprising:

   a step of measuring the TG reactivity indices of single-coal cokes and estimating the TG reactivity index of the corresponding coal-blend coke from the weighted average of the TG reactivity indices measured of the single-coal cokes;
   a step of estimating a coke reactivity index (CRI) of the coal-blend coke from the TG reactivity index of the coal-blend coke estimated in the previous step; and
   a step of estimating a coke strength of the coal-blend coke after $CO_2$ reaction (CSR) from the coke reactivity index (CRI) of the coal-blend coke estimated in the previous step and a drum index (DI) of the coal-blend coke.

2. The coal-blend coke CSR estimating method according to claim 1, wherein the thermogravimetry is performed by heating the coke in an inert gas atmosphere to a temperature range from 950°C to 1100°C.

3. The coal-blend coke CSR estimating method according to claim 1, wherein the predetermined time is 10 minutes or more and 120 minutes or less.

4. The coal-blend coke CSR estimating method according to claim 2, wherein the predetermined time is 10 minutes or more and 120 minutes or less.

5. A method for producing coal-blend coke, comprising:

   estimating a coke strength of coal-blend coke after $CO_2$ reaction using the coal-blend coke CSR estimating method described in any one of claims 1 to 4; and
   changing a production condition based on the estimated coke strength of coal-blend coke after $CO_2$ reaction.

6. A method of operating a coke oven, comprising:

   estimating a coke strength of coal-blend coke after $CO_2$ reaction using the coal-blend coke CSR estimating method described in any one of claims 1 to 4; and
   changing a coke oven operation condition based on the estimated coke strength of coal-blend coke after $CO_2$ reaction.

7. A method for controlling blending ratios of single coals, comprising:

**EP 4 663 718 A1**

estimating a coke strength of coal-blend coke after $CO_2$ reaction using the coal-blend coke CSR estimating method described in any one of claims 1 to 4; and
changing blending ratios of single coals based on the estimated coke strength of coal-blend coke after $CO_2$ reaction.

# FIG. 1

# FIG. 2

# FIG. 3

# FIG. 4

**EP 4 663 718 A1**

## INTERNATIONAL SEARCH REPORT

<table>
<tr><td colspan="2">International application No.</td></tr>
<tr><td colspan="2"><b>PCT/JP2024/005707</b></td></tr>
</table>

**A. CLASSIFICATION OF SUBJECT MATTER**

*C10B 57/00*(2006.01)i; *C10B 57/04*(2006.01)i
FI: C10B57/00; C10B57/04

According to International Patent Classification (IPC) or to both national classification and IPC

**B. FIELDS SEARCHED**

Minimum documentation searched (classification system followed by classification symbols)

C10B57/00; C10B57/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2013-1895 A (NIPPON STEEL & SUMITOMO METAL CORPORATION) 07 January 2013 (2013-01-07) entire text | 1-7 |
| A | JP 2005-232350 A (NIPPON STEEL CORPORATION) 02 September 2005 (2005-09-02) entire text | 1-7 |
| A | JP 9-263766 A (NIPPON STEEL CORPORATION) 07 October 1997 (1997-10-07) entire text | 1-7 |
| P, A | WO 2023/100600 A1 (JFE STEEL CORPORATION) 08 June 2023 (2023-06-08) entire text | 1-7 |

☐ Further documents are listed in the continuation of Box C.   ☑ See patent family annex.

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 April 2024** | **14 May 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

## INTERNATIONAL SEARCH REPORT
### Information on patent family members

International application No.

**PCT/JP2024/005707**

| Patent document cited in search report | | | Publication date (day/month/year) | Patent family member(s) | Publication date (day/month/year) |
|---|---|---|---|---|---|
| JP | 2013-1895 | A | 07 January 2013 | (Family: none) | |
| JP | 2005-232350 | A | 02 September 2005 | (Family: none) | |
| JP | 9-263766 | A | 07 October 1997 | (Family: none) | |
| WO | 2023/100600 | A1 | 08 June 2023 | (Family: none) | |

Form PCT/ISA/210 (patent family annex) (July 2022)

# EP 4 663 718 A1

**Patent documents cited in the description**

- JP 63199286 A
- JP 9263766 A
- JP 2005232350 A
- JP 2013001895 A